# FASCICULE DE BREVET EUROPEEN

(11) **EP 3 298 158 B1**
(45) Date de publication et mention de la délivrance du brevet: **10.07.2019**
(21) Numéro de dépôt: 16730879.0
(22) Date de dépôt: 19.05.2016
(51) Int. Cl.: C12Q 1/18, C12Q 1/04, A61K 31/397

(54) **IDENTIFICATION DES BACTÉRIES RESISTANTES AUX CARBAPÉNÈMES PAR IMPERMEABILITÉ MEMBRANAIRE**
IDENTIFIZIERUNG VON RESISTENTEN BAKTERIEN GEGEN CARBAPENEME DURCH MEMBRANUNDURCHLÄSSIGKEIT
IDENTIFICATION OF BACTERIA THAT ARE RESISTANT TO CARBAPENEMS BY MEMBRANE IMPERMEABILITY

(30) Priorité: 20.05.2015 FR 1554522
(43) Date de publication de la demande: 28.03.2018
(73) Titulaire: Biomérieux, 69280 Marcy-L'Etoile (FR); Institut National de la Santé et de la Recherche Médicale (I.N.S.E.R.M.), 75654 Paris Cedex 13 (FR); Université d'Aix Marseille, 13284 Marseille Cedex 07 (FR); Service de Santé des Armées (SSA), 75614 Paris Cedex 12 (FR)
(72) Inventeur: FRANCESCHI, Christine, 01800 Meximieux (FR); PINET, Elizabeth, 17137 Esnandes (FR); PAGES, Jean-Marie, 13005 Marseille (FR); ZAMBARDI, Gilles, 38230 Tignieu (FR)
(74) Mandataire: Sprugnoli, Claude Louis Victor
(86) Numéro de dépôt international: PCT/FR2016/051180
(87) Numéro de publication internationale: WO 2016/185141

(56) Documents cités:
- NIKAIDO H: "The role of outer membrane and efflux pumps in the resistance of gram-negative bacteria. Can we improve drug access?", DRUG RESISTANCE UPDATES, CHURCHILL LIVINGSTONE, EDINBURGH, GB, vol. 1, no. 2, 1 janvier 1998 (1998-01-01) , pages 93-98, XP004969739, ISSN: 1368-7646, DOI: 10.1016/S1368-7646(98)80023-X
- X. Z. LI ET AL: "Role of mexA-mexB-oprM in antibiotic efflux in Pseudomonas aeruginosa", ANTIMICROBIAL AGENTS AND CHEMOTHERAPY, vol. 39, no. 9, 1 septembre 1995 (1995-09-01), pages 1948-1953, XP055255836, US ISSN: 0066-4804, DOI: 10.1128/AAC.39.9.1948
- P VILJANEN ET AL: "Susceptibility of gram-negative bacteria to polymyxin B nonapeptide.", ANTIMICROBIAL AGENTS AND CHEMOTHERAPY, vol. 25, no. 6, 1 juin 1984 (1984-06-01), pages 701-705, XP055255839, US ISSN: 0066-4804, DOI: 10.1128/AAC.25.6.701
- JIA CHANG CAI ET AL: "Detection of OmpK36 Porin Loss in Klebsiella spp. by Matrix-Assisted Laser Desorption Ionization-Time of Flight Mass Spectrometry", JOURNAL OF CLINICAL MICROBIOLOGY, vol. 50, no. 6, 4 avril 2012 (2012-04-04), pages 2179-2182, XP055255921, US ISSN: 0095-1137, DOI: 10.1128/JCM.00503-12 cité dans la demande
- CHAMPLIN F R ET AL: "Effect of outer membrane permeabilisation on intrinsic resistance to low triclosan levels in Pseudomonas aeruginosa", INTERNATIONAL JOURNAL OF ANTIMICROBIAL AGENTS, ELSEVIER SCIENCE, AMSTERDAM, NL, vol. 26, no. 2, 1 août 2005 (2005-08-01), pages 159-164, XP027807458, ISSN: 0924-8579 [extrait le 2005-08-01]
- KEN EGUCHI ET AL: "The Mode of Action of 2-(Thiazol-2-ylthio)-1[beta]-methylcarbape nems against Pseudomonas aeruginosa: The Impact of Outer Membrane Permeability and the Contribution of MexAB-OprM Efflux System", THE JOURNAL OF ANTIBIOTICS, vol. 60, no. 2, 1 février 2007 (2007-02-01), pages 129-135, XP055255726, GB ISSN: 0021-8820, DOI: 10.1038/ja.2007.12
- M VAARA: "Antimicrobial susceptibility of Salmonella typhimurium carrying the outer membrane permeability mutation SS-B.", ANTIMICROBIAL AGENTS AND CHEMOTHERAPY, vol. 34, no. 5, 1 mai 1990 (1990-05-01), pages 853-857, XP055256211, US ISSN: 0066-4804, DOI: 10.1128/AAC.34.5.853
- JAMES H. JORGENSEN ET AL: "Antimicrobial Susceptibility Testing: A Review of General Principles and Contemporary Practices", CLINICAL INFECTIOUS DISEASES, vol. 49, no. 11, 1 décembre 2009 (2009-12-01), pages 1749-1755, XP055034346, ISSN: 1058-4838, DOI: 10.1086/647952
- DELCOUR ET AL: "Outer membrane permeability and antibiotic resistance", BIOCHIMICA ET BIOPHYSICA ACTA (BBA) - PROTEINS & PROTEOMICS, ELSEVIER, NETHERLANDS, vol. 1794, no. 5, 1 mai 2009 (2009-05-01), pages 808-816, XP026090178, ISSN: 1570-9639, DOI: 10.1016/J.BBAPAP.2008.11.005 [extrait le 2008-11-27]

## Description

La présente invention appartient au domaine technique de la microbiologie clinique, et a trait aux techniques de dépistage et de diagnostic des maladies infectieuses. Elle concerne plus particulièrement le profilage des bactéries résistantes aux antibiotiques et à l'identification des mécanismes moléculaires impliqués dans cette résistance.

Les antibiotiques sont employés à des fins médicales depuis la Seconde Guerre Mondiale. Du fait d'une utilisation massive, répétée et parfois inconsidérée, tant au niveau thérapeutique qu'au niveau industriel (notamment pour le traitement préventif des animaux d'élevage destinés à l'alimentation), ces molécules et leur efficacité thérapeutique se trouvent aujourd'hui menacées de déclin par l'émergence et l'expansion de souches bactériennes de plus en plus résistantes.

En vue de minimiser les diagnostiques erronés et les errances thérapeutiques, les industriels du domaine de la santé oeuvrent pour assister les praticiens dans leur fonction, en mettant à leur disposition des méthodes et outils analytiques de plus en plus performants pour détecter et identifier les microorganismes infectieux.

La présente invention s'inscrit dans le développement de tels méthodes et outils analytiques et, plus spécifiquement, ceux qui sont dédiés au profilage des bactéries qui sont dites résistantes aux antibiotiques. Plus précisément, elle concerne les méthodes et les outils qui ont vocation à caractériser le type de mécanisme moléculaire développé par certaines bactéries pour réduire leur sensibilité aux antibiotiques.

Les mécanismes de résistance aux antibiotiques sont multiples. De façon générale et schématique, ils peuvent être classés selon les catégories de résistance suivantes :
- mutation ou surexpression de la cible de l'antibiotique,
- inactivation enzymatique de l'antibiotique,
- réduction de la perméabilité de la paroi cellulaire,
- modification de l'efflux actif des antibiotiques,
- formation d'un biofilm.

La prévalence de ces mécanismes diffère d'une bactérie à une autre, mais également d'un type d'antibiotique à un autre.

Dans le cas des antibiotiques de la famille des carbapénèmes (tels que l'imipénème, le méropénème, l'ertapénème, le doripénème, le tébipénème, le faropénème), les bactéries ont principalement développé deux types de résistance :
1) une résistance par hydrolyse de l'antibiotique, qui se caractérise par la production d'enzymes à activité carbapénémase, et
2) une résistance par réduction de la perméabilité membranaire ; on parle aussi d'imperméabilité membranaire.

La présente invention s'intéresse plus particulièrement à la sensibilité/résistance des bactéries aux carbapénèmes. Plus spécifiquement, elle vise à déterminer si une résistance aux carbapénèmes développée par une bactérie peut oui ou non être attribuée à une réduction de sa perméabilité membranaire. Autrement dit, elle vise à distinguer/discriminer parmi les bactéries présentant une résistance aux carbapénèmes, celles qui le sont grâce à un mécanisme de résistance par imperméabilité membranaire.

Plusieurs méthodes analytiques tendant vers ce même objectif ont déjà été décrites dans l'état de l'art. La plupart cible les porines, des protéines membranaires constitutives de canaux qu'empruntent les antibiotiques (ainsi que d'autres molécules) pour pénétrer dans les bactéries. Un défaut d'expression des porines est ainsi utilisé comme un indicateur d'une résistance aux antibiotiques par imperméabilité membranaire.

Pour leur mise en oeuvre, ces méthodes font appel notamment à des techniques d'immuno-détection (test ELISA, Western blot), de biologie moléculaire (Southern blot, Northern blot, séquençage de séquence nucléiques), de spectrométrie de masse, notamment la spectrométrie de masse MALDI-TOF (Cai et al., JOURNAL OF CLINICAL MICROBIOLOGY (2012), 50(6): 2179-82), en vue soit d'une détection physique des porines, soit d'une évaluation de la capacité des bactéries testées à exprimer ces protéines.

Ces méthodes analytiques, qui ciblent les porines, présentent un inconvénient majeur ; les porines existent sous de nombreuses isoformes (notamment Omp1, Omp2, Omp35, Omp36, Omp37, OmpC, OmpD, OmpF, OmpN, OmpK35, OmpK36, OmpK37...) dont l'occurrence dépend et est fonction du genre bactérien et de l'espèce bactérienne. Ainsi, à moins de disposer d'anticorps, de sondes nucléiques et/ou de spectres SM de référence permettant la détection/identification de la totalité des isoformes de porines existants, une mise en oeuvre fiable de ces méthodes nécessite non seulement une identification préalable et précise du genre bactérien à analyser, mais également, une connaissance exacte des isoformes particulières exprimées dans ce genre bactérien, et de disposer d'anticorps, de sondes nucléiques et/ou de spectres SM de référence appropriés à la détection/identification de ces isoformes particulières.

Un autre inconvénient, qui joue également sur la fiabilité de ces méthodes, réside dans le fait qu'une détection positive de l'expression des porines ne permet pas d'écarter de façon certaine une possible résistance par imperméabilité membranaire. En effet, il faudrait pour cela vérifier également que cette expression des porines soit à un niveau suffisant et s'assurer que les protéines exprimées soient assemblées de manière fonctionnelle dans la membrane.

La présente invention vise à pallier les désagréments, les désavantages et la lourdeur rencontrés avec les méthodes antérieures. En particulier, elle vise à proposer une méthode de détection et/ou d'identification de bactéries ayant développé une résistance aux carbapénèmes par imperméabilité membranaire, qui puisse s'affranchir de la grande variabilité des isoformes des porines et de la nécessité d'une vérification de leur expression à un niveau fonctionnel et opérationnel.

Un autre objectif de la présente invention consiste à proposer une méthode analytique qui puisse être de mise en oeuvre relativement simple et rapide, et ne nécessitant pas d'installations techniques lourdes, sophistiquées et coûteuses (telles que par exemple du matériel de spectroscopie de masse...).

Un autre objectif de la présente invention consiste à proposer une méthode et des outils associés qui puissent être adaptés et applicables à un grand nombre de genres, d'espèces et de souches bactériennes.

La présente invention propose ainsi une méthode de discrimination de bactéries résistantes aux carbapénèmes, selon qu'elles développent ou non une résistance par imperméabilité membranaire, ladite méthode comprenant les étapes suivantes :
- disposer d'une souche bactérienne résistante à au moins un carbapénème,
- évaluer la sensibilité de ladite souche bactérienne à l'égard d'au moins un carbapénème indicateur ; d'une part, en absence de polymyxine B nonapeptide (PMBN) et, d'autre part, en présence de PMBN à une concentration inférieure à la concentration minimale inhibitrice (CMI) de la PMBN pour ladite souche bactérienne, dite concentration non-inhibitrice de PMBN;
- poser un pronostic de résistance par imperméabilité membranaire dans le cas où, la présence de PMBN permet d'accroitre de manière significative la sensibilité de ladite souche bactérienne à l'égard d'au moins un carbapénème indicateur utilisé.

Dans le cas où il est constaté que la présence de PMBN ne permet pas d'accroitre de manière significative la sensibilité de ladite souche bactérienne au(x)dit(s) carbapénème(s) indicateur(s), un pronostic de résistance autre qu'une imperméabilité membranaire est alors posé. Il s'agit alors très vraisemblablement d'une résistance par production d'enzymes à activité carbapénémase.

Dans le cadre de la conception de la présente invention, il a été expressément mis en évidence qu'une bactérie résistante aux carbapénèmes par imperméabilité membranaire, voit sa sensibilité aux carbapénèmes croître de façon significative, lorsqu'elle est traitée par un agent perméabilisant particulier et ce, à des concentrations particulières de cet agent perméabilisant. *A contrario*, ce même traitement de perméabilisation, lorsqu'il est appliqué à une bactérie développant une autre forme de résistance, ne produit nullement un tel effet.

La présente invention propose ainsi une méthode de discrimination de bactéries ayant une résistance par imperméabilité membranaire, dont le principe consiste à constater qu'il est effectivement possible de conférer à ces bactéries un niveau de perméabilité apte à rétablir chez elles une « sensibilité naturelle » aux carbapénèmes.

Ainsi, pour chaque bactérie testée, la mise en oeuvre de la méthode de discrimination selon l'invention permet l'obtention d'une information relative à la sensibilité de la bactérie à un carbapénème indicateur, une sensibilité évaluée en absence et en présence d'une concentration non-inhibitrice de PMBN. En fonction de cette information, un pronostic est posé quant à la nature du mécanisme de résistance développé par la bactérie.

Au-delà de l'originalité du concept qui sous-tend la méthode proposée, il est également du mérite des inventeurs d'avoir identifié, chez la PMBN, des propriétés de perméabilisation membranaire particulièrement adaptées à la concrétisation de cette méthode et d'avoir ainsi détourné l'utilisation de cette molécule, initialement développée pour un usage exclusivement thérapeutique, pour en faire un outil analytique.

En effet, jusqu'à présent, la PMBN est connue comme étant un antibiotique dérivé de la polymyxine B. Comme toute polymyxine, il s'agit d'un peptide cyclique doté d'une queue hydrophobe, et dont l'action antibiotique se traduit par une déstructuration dénaturante de la paroi bactérienne. La queue amino-acyl de la PMBN est plus courte que celle de la polymixine B.

Comme toute polymyxine ou autre agent perméabilisant membranaire, son utilisation thérapeutique combinée à d'autres antibiotiques a également été envisagée (WO 2008/017734). Ce faisant, outre une accumulation des effets thérapeutiques de chacun des antibiotiques utilisés (y compris la PMBN), une meilleure pénétration des antibiotiques autres que la PMBN est également recherchée.

Dans le cadre de la présente invention, les inventeurs ont su mettre à profit le pouvoir perméabilisant relativement « modéré » de cette molécule ; un pouvoir perméabilisant modéré qui la rend adaptée à une utilisation à des concentrations non-inhibitrices dans des compositions complexes, tels que des milieux de culture. Les quantités de PMBN nécessaires à l'obtention de telles concentrations restent en effet compatibles avec les techniques traditionnelles de mélange et de formulation de compositions complexes, homogènes tant au niveau chimique que biologique.

Dans le cadre de la présente invention, le caractère significatif de l'accroissement de la sensibilité aux carbapénèmes peut être laissé à l'appréciation de l'opérateur. Celui-ci, à l'instar de tout homme du métier, est à même de distinguer une augmentation de sensibilité dont l'amplitude est suffisamment importante pour l'attribuer/imputer à un effet de la PMBN, plutôt qu'à un « bruit de fond » et/ou à des incertitudes de mesure ou d'interprétation qui affectent les méthodes traditionnelles d'évaluation de sensibilité des bactéries aux antibiotiques.

Plus objectivement, au sens de la présente invention, il est considéré que l'accroissement de la sensibilité d'une souche bactérienne à un carbapénème indicateur est significatif dès lors qu'il est constaté une diminution de la CMI correspondante (mesurée ou évaluée) d'au moins un facteur 2, voire d'au moins un facteur 3-4.

Avant d'aller plus loin dans la description de l'invention, les définitions ci-après sont données afin de faciliter la compréhension et l'exposé de l'invention.

Par « milieu de culture », on entend un milieu comprenant les éléments nécessaires à la survie et la multiplication de microorganismes, en l'occurrence des bactéries. La composition d'un milieu de culture peut comprendre divers constituants tels que : acides aminés, peptones, hydrates de carbone, nucléotides, minéraux, vitamines, facteurs de croissance (par exemple du sang de mouton, de cheval)... et, éventuellement, colorants (par exemple, le bleu d'Evans, du rouge neutre), opacifiants (par exemple, l'oxyde de titane), indicateurs métaboliques, régulateurs métaboliques...

Un milieu de culture peut être solide, semi-solide ou liquide.

Par « milieu de culture solide » ou « milieu solide », on entend par exemple un milieu gélifié. L'agar est l'agent gélifiant traditionnel en microbiologie pour la culture des microorganismes, mais il est également possible d'utiliser de la gélatine, de l'agarose ou d'autres gélifiants naturels ou artificiels. Les milieux de culture disponibles dans le commerce sont nombreux, comme par exemple l'agar Columbia, la gélose Trypcase-soja, la gélose Mac Conkey, la gélose Mueller Hinton ou plus généralement celles décrites dans le Handbook of Microbiological Media (CRC Press).

Par « bactérie résistante aux carbapénèmes » ou plus simplement « bactérie résistante », on entend une bactérie ayant une résistance avérée pour au moins un carbapénème, peu importe le mécanisme de résistance développé à l'égard de cet antibiotique.

Par « concentration non-inhibitrice de PMBN », on entend une concentration de PMBN produisant sur une bactérie une déstructuration/dénaturation de la membrane qui ne soit pas de nature à lyser la bactérie ou à inhiber sa multiplication. Objectivement, au sens de la présente invention, cette concentration « non-inhibitrice » ou « sub-inhibitrice » est inférieure à la concentration minimale inhibitrice (CMI) de la PMBN pour ladite bactérie.

Par « carbapénème indicateur », on entend un antibiotique appartenant à la classe des carbapénèmes, qui est utilisé pour la mise en oeuvre de la présente invention et à l'égard duquel la susceptibilité/sensibilité de la bactérie à tester est évaluée. Le carbapénème choisi comme carbapénème indicateur, au sens de la présente invention, ne correspond pas nécessairement au carbapénème ou à l'un des carbapénèmes pour le(s)quel(s) la bactérie à tester a développé une résistance avérée. Il est préférentiellement choisi pour son large spectre d'action, sa bonne stabilité chimique et enzymatique. Avantageusement et selon l'invention, chaque carbapénème indicateur est préférentiellement choisi parmi : l'imipénème, l'ertapénème et le méropénème.

Selon un mode de mise en oeuvre particulier de l'invention, la sensibilité de la souche bactérienne à tester est évaluée à l'égard d'un carbapénème indicateur unique. Une résistance par imperméabilité membranaire est donc attribuée à cette souche bactérienne dès lors qu'il est constaté que la présence de PMBN permet d'accroitre de manière significative la sensibilité de cette souche bactérienne à ce carbapénème indicateur particulier.

Selon un autre mode de mise en oeuvre de l'invention, la sensibilité de la souche bactérienne à tester est évaluée à l'égard de plusieurs carbapénèmes indicateurs, de préférence deux. Dans ce cas, un pronostic de résistance par imperméabilité membranaire est posé dès lors qu'il est constaté que la présence de PMBN permet d'accroitre de manière significative la sensibilité de cette souche bactérienne à l'égard d'au moins un des carbapénèmes indicateurs utilisés.

S'agissant de la concentration non-inhibitrice de PMBN à utiliser pour mettre en oeuvre la présente méthode de discrimination, celle-ci peut être choisie de façon spécifique par rapport à la souche bactérienne à tester, ou de façon non-spécifique si la souche n'est pas identifiée.

Lorsqu'elle est choisie de façon spécifique, ladite concentration non-inhibitrice de PMBN à utiliser découle de la valeur de la CMI de la PMBN préalablement déterminée/évaluée pour la souche bactérienne à tester. Avantageusement et selon l'invention, ladite concentration non-inhibitrice de PMBN est comprise entre de l'ordre de un dixième (1/10) et de l'ordre de un demi (1/2) de la CMI de la PMBN pour ladite souche bactérienne. Elle est plus préférentiellement choisie entre un huitième (1/8) et un cinquième (1/5) de ladite CMI.

La détermination/évaluation préalable de la CMI de la PMBN peut être réalisée par toute méthode appropriée connue de l'homme du métier, par exemple par :
- une méthode de dilution (ou de micro-dilution), en milieu solide ou en milieu liquide,
- une méthode de diffusion dans laquelle est utilisé un disque d'antibiotique ou une bandelette de gradient d'antibiotique, imprégnés de PMBN.

Lorsque la concentration non-inhibitrice de PMBN à utiliser pour mettre en oeuvre la présente invention est choisie de façon non-spécifique, par rapport à la souche bactérienne à tester, il devient inutile de déterminer/évaluer au préalable la CMI de la PMBN pour cette souche bactérienne. Avantageusement et selon l'invention, on choisira alors une concentration de PMBN « standard », avantageusement comprise entre 25 µg/mL et 500 µg/mL, préférentiellement comprise entre de l'ordre de 150 µg/mL et de l'ordre 350 µg/mL. Pour la plupart des bactéries, en particulier les entérobactéries, de telles concentrations correspondent effectivement à des concentrations non-inhibitrices de PMBN au sens de la présente invention.

S'agissant de l'évaluation de la sensibilité de la souche bactérienne à chaque carbapénème indicateur, cette étape peut être réalisée par toute méthode appropriée connue de l'homme du métier.

Selon un premier mode de réalisation, cette étape est avantageusement réalisée au moyen d'une méthode de diffusion sur milieu (de culture) solide. Pour ce faire, chaque carbapénème indicateur est utilisé sous la forme d'un disque d'antibiotique ou sous la forme d'une bandelette de gradient d'antibiotique, notamment des bandelettes Etest® (bioMérieux, France).

Selon ce premier mode préféré de réalisation, lorsque l'évaluation de la sensibilité d'une souche bactérienne à un carbapénème indicateur est réalisée en présence de PMBN, la PMBN est initialement présente dans ledit milieu solide à une concentration non-inhibitrice.

Selon une variante de réalisation, la PMBN n'est pas initialement présente dans ledit milieu solide, mais y est apportée de façon extemporanée et concomitante avec le(s) carbapénème(s) indicateur(s). Pour ce faire, avantageusement et selon l'invention, on utilise des disques d'antibiotique ou des bandelettes de gradient d'antibiotique, imbibés à la fois d'un carbapénème indicateur et de PMBN. Avantageusement et selon un mode de réalisation particulier, s'agissant de la PMBN, lesdits disques et lesdites bandelettes ont été préalablement préparés par imprégnation avec une solution de PMBN de concentration en PMBN comprise entre de l'ordre de 300 µg/mL et de l'ordre de 500 µg/mL.

Selon un second mode préféré de réalisation de l'étape d'évaluation de la sensibilité d'une souche bactérienne à l'égard d'un carbapénème indicateur, on utilise une méthode de micro-dilution en milieu liquide. Pour ce faire, un inoculum standardisé de la souche bactérienne à tester est mis au contact d'une série de réceptacles (tels que par exemple, des tubes ou des cupules) renfermant un milieu de culture liquide avec une concentration croissante d'un carbapénème indicateur. Après incubation, la CMI est donnée par le réceptacle qui contient la plus faible concentration d'antibiotique et dans lequel aucune croissance bactérienne n'est visible. Lorsque cette évaluation est réalisée en présence de PMBN, chaque réceptacle de milieu de culture contient également une concentration non-inhibitrice de PMBN.

Cette concentration non-inhibitrice de PMBN peut être identique d'un réceptacle à l'autre. Selon une variante de réalisation, elle varie avantageusement d'un réceptacle à l'autre et, pour un réceptacle donné, sa valeur est indexée sur la concentration du carbapénème indicateur du milieu de culture contenu dans ce réceptacle.

Avantageusement et selon l'invention, l'évaluation de la sensibilité de la souche bactérienne par la méthode de micro-dilution en milieu liquide est réalisée de façon automatisée, par exemple en adaptant des automates, par exemple le VITEK® 2 AST (bioMérieux, France).

Est aussi décrit un nécessaire (ou kit) permettant la mise en oeuvre d'une méthode de discrimination de bactéries résistantes aux carbapénèmes selon qu'elles présentent ou non une résistance par imperméabilité membranaire.

Selon un premier aspect, un nécessaire comprend au moins :
- un milieu de culture solide exempt de PMBN,
- un milieu de culture solide comprenant une concentration non-inhibitrice de PMBN, et
- deux disques d'antibiotique ou deux bandelettes de gradient d'antibiotique, imbibés d'un carbapénème indicateur.

Selon un deuxième aspect, un nécessaire comprend au moins :
- un milieu de culture solide exempt de PMBN,
- deux disques d'antibiotique ou deux bandelettes de gradient d'antibiotique, imbibés d'un carbapénème indicateur,
- deux disques d'antibiotique ou deux bandelettes de gradient d'antibiotique, imbibés à la fois d'un carbapénème indicateur et de PMBN.

Selon un troisième aspect, un nécessaire comprend au moins :
- une première série de réceptacles, dans laquelle chaque réceptacle renferme un milieu de culture exempt de PMBN et comprenant une concentration d'un carbapénème indicateur, les concentrations en carbapénème indicateur des différents réceptacles formant une gamme de concentrations ;
- une deuxième série de réceptacles, dans laquelle chaque réceptacle renferme un milieu de culture, comprenant une concentration d'un carbapénème indicateur et une concentration non-inhibitrice de PMBN, les concentrations en carbapénème indicateur des différents réceptacles formant une gamme de concentrations et les concentrations non-inhibitrices de PMBN des différents réceptacles étant identiques les unes aux autres ou indexées sur la concentration en carbapénème indicateur du milieu de culture contenu dans le réceptacle considéré.

Ce document s'étend également à une méthode de discrimination de bactéries résistantes aux carbapénèmes selon qu'elles développent ou non une résistance par imperméabilité membranaire, une méthode permettant de pronostiquer une résistance aux carbapénèmes par imperméabilité membranaire, ainsi qu'un nécessaire (ou kit) permettant la mise en oeuvre de cette méthode de discrimination et/ou de pronostic, caractérisés par tout ou partie des caractéristiques techniques exposées ci-dessus et ci-dessous.

D'autres buts, caractéristiques et avantages de l'invention apparaîtront au vu de la description qui va suivre et des exemples, développés ci-dessous, qui ont pour but de faciliter la compréhension de l'invention et de sa mise en oeuvre. Ces exemples sont donnés à titre explicatif et ne sauraient limiter la portée de l'invention.

### EXEMPLES

### 1. Mise au point de la méthode selon l'invention sur des souches bactériennes de phénotype connu (« porine + » ou « porine - »)

Dans un premier temps, des souches bactériennes résistantes aux carbapénèmes ayant un phénotype « porine + » et « porine - », ont été utilisées comme souches modèles pour évaluer la pertinence de la méthode de discrimination selon l'invention, et pour caractériser les conditions opératoires appropriées à sa mise en oeuvre. Il s'agissait en particulier de tester différentes concentrations non-inhibitrices de PMBN et divers carbapénèmes à titre de carbapénèmes indicateurs.

### a) Souches modèles utilisées

Les souches bactériennes utilisées dans cet essai proviennent de collections appartenant aux titulaires du présent Titre de propriété industrielle. Ces collections ont été constituées au fil des années et rassemblent des souches de microorganismes d'origines diverses (prélèvements cliniques, collections internationales, et autres).

Ces souches résistantes ont été testées au préalable par rapport à la présence ou non de porines dans leur membrane. Pour ce faire, des analyses en Western blot ont été réalisées pour détecter la présence des isoformes OmpF et OmpC, deux isoformes connues pour être largement exprimées par le genre bactérien auquel appartiennent ces souches.

Une souche est ainsi notée « porine - », lorsqu'aucune des deux isoformes précédemment citées n'a pu être détectée. Une résistance par imperméabilité membranaire est alors fortement suspectée.

Une souche est par contre notée « porine + », lorsqu'au moins une des deux isoformes précédemment citées a pu être détectée. Un mécanisme de résistance autre qu'une imperméabilité membranaire est alors envisagé pour cette souche.

Egalement, préalablement à la mise en oeuvre de la méthode de discrimination selon l'invention, la sensibilité à la PMBN de chacune des souches modèles utilisées a été évaluée au moyen d'une méthode de micro-dilution en milieu liquide.

Pour ce faire, chacune des souches a été cultivée en bouillon Mueller-Hinton (MHII - BECTON DICKINSON®, Etats-Unis). Sur des inocula contenant 2.10⁵ UFC/mL, des dilutions successives de 2 en 2 ont été réalisées pour la PMBN afin de déterminer les concentrations minimales inhibitrices. La lecture de la sensibilité à la PMBN a été réalisée après 18 heures d'incubation à 37°C, par addition de 0,2 mg/mL d'iodonitrotétrazolium chloride (SIGMA-ALDRICH®, Etats-Unis).

Dans le Tableau 1 ci-après, sont listées les souches modèles utilisées, leur phénotype constaté, ainsi que leur CMI à la PMBN.

**Tableau 1**

| Souches testées | Phénotype | CMI_{PMBN} (µg/mL) |
|---|---|---|
| *Enterobacter aerogenes* EA 2 | porine + | 1024 |
| *Enterobacter aerogenes* EA 5 | porine - | 1024 |
| *Enterobacter aerogenes* EA 27 | porine - | 256 |
| *Enterobacter aerogenes* EA 15038 | porine + | 256 |

### b) Méthode mise en oeuvre avec différents carbapénèmes indicateurs et différentes concentrations non-inhibitrices de PMBN

Dans un premier temps, deux carbapénèmes ont été testés à titre de carbapénèmes indicateurs : l'imipénème (IM) et l'ertapénème (ETP).

Pour chaque souche modèle, la CMI à chacun de ces deux carbapénèmes a été déterminée en absence et en présence de PMBN (à des concentrations non-inhibitrices).

Les déterminations de CMI aux antibiotiques ont été réalisées sur des milieux de culture solides avec la méthode Etest® . Pour ce faire, ont été utilisées des bandelettes Etest® (bioMérieux, France) :
- Etest® Imipenem IM 32, et
- Etest® Ertapenem ETP 32,
ainsi que des milieux de culture solides, différant les uns des autres par leurs concentrations non-inhibitrices en PMBN.

Ces milieux de culture solides sont réalisés en boîtes de Petri (Ø = 145 mm ; V = 50 mL) sur une base de gélose d'agar MH II (BECTON DICKINSON®, Etats-Unis), plus ou moins complémentée en PMBN. Les concentrations en PMBN vont de 0 jusqu'à des concentrations équivalentes à un cinquième de la CMI de la PMBN (1/5 CMI_{PMBN}).

A l'aide d'un écouvillon et de l'ensemenceur rotatif Retro C80™ (bioMérieux, France), les milieux de culture ont été ensemencés avec les bactéries, provenant d'un inoculum de 0,5 Mac Farland, préparé en solution NaCl à 0,85 % à partir de colonies repiquées la veille sur gélose MH II.

Les différentes bandelettes Etest® sont alors déposées sur la surface des milieux de culture. Après 16 à 20 heures d'incubation à 35°C, la lecture des CMI a été effectuée.

Le Tableau 2 ci-après résume les résultats obtenus (les CMI y sont exprimées en µg/mL).

**Tableau 2**

| | ***Enterobacter aerogenes* EA 2** (porine +) CMI_{PMBN} = 1024 µg/ml | | | | ***Enterobacter aerogenes* EA 15038** (porine +) CMI_{PMBN} = 256 µg/ml | | | |
|---|---|---|---|---|---|---|---|---|
| [*PMBN*] | *0* | *CMI*/*10* | *CMI*/*8* | *CMI*/*5* | *0* | *CMI*/*10* | *CMI*/*8* | *CMI*/*5* |
| CMI_{IM} | 0,03 | 0,06 | 0,06 | 0,03 | 0,25 | 0,5 | 0,5 | 0,25 |
| CMI_{ETP} | 0,03 | 0,25 | 0,25 | 0,125 | 0,25 | 0,125 | 0,125 | 0,06 |
| | | | | | | | | |

| | ***Enterobacter aerogenes* EA 27** (porine -) CMI_{PMBN} = 1024 µg/mL | | | | ***Enterobacter aerogenes* EA 5** (porine-) CMI_{PMBN} = 1024 µg/ml | | | |
|---|---|---|---|---|---|---|---|---|
| [*PMBN*] | *0* | *CMI*/*10* | *CMI*/*8* | *CMI*/*5* | *0* | *CMI*/*10* | *CMI*/*8* | *CMI*/*5* |
| CMI_{IM} | 8 | 8 | 0,06 | 0,03 | 4 | 2 | 2 | 2 |
| CMI_{ETP} | 64 | 16 | 0,25 | 0,125 | 32 | 4 | 4 | 4 |

Au vu de ces premiers résultats, il apparait que les bactéries résistantes aux carbapénèmes, exposées à une concentration non-inhibitrice de PMBN, répondent différemment à l'imipénème (IM) et l'ertapénème (ETP), en fonction du mécanisme de résistance qu'elles ont développé à l'égard des carbapénèmes.

A l'exception des résultats obtenus avec la souche EA 15038 évaluée avec l'ertapénème, il est ainsi constaté que les souches ayant développé une résistance par imperméabilité membranaire (souches « porine - ») voient leur sensibilité aux carbapénèmes croître de façon significative en présence d'une concentration non-inhibitrice de PMBN (reflétée par une baisse des CMI_{IM} et CMI_{ETP}). A l'inverse, pour les souches ayant développé une autre forme de résistance, leurs CMI_{IM} et CMI_{ETP} restent inchangées ou ont tendance à augmenter.

Ces premiers résultats confirment le potentiel qu'il existe à utiliser en combinaison une concentration non-inhibitrice de PMBN et d'un carbapénème indicateur pour pronostiquer/identifier le mécanisme de résistance aux carbapénèmes développé par une bactérie, notamment une entérobactérie.

### 2. Validation et optimisation de la méthode selon l'invention

Dans un deuxième temps, la méthode de discrimination selon l'invention a été mise en oeuvre, « à l'aveugle », sur diverses souches bactériennes résistantes aux carbapénèmes (parmi ces souches, certaines ont été préalablement identifiées comme productrices de carbapénémases).

Pour ce faire, à titre de carbapénèmes indicateurs, l'imipénème et l'ertapénème ont été utilisés. Des concentrations non-inhibitrices de PMBN, équivalentes à des concentrations de un dixième (1/10), un huitième (1/8) et un cinquième (1/5) de la CMI de PMBN, ont été appliquées.

Pour chaque souche testée, chaque concentration de PMBN et chaque carbapénème indicateur, la CMI mesurée est comparée à celle obtenue en l'absence de PMBN. Au vu de l'écart éventuel de valeurs de CMI, le mécanisme de résistance développé par la souche testée est pronostiqué en appliquant le postulat général suivant : Une diminution de CMI_{IM/ETP} en présence de PMBN est l'indice d'une résistance aux carbapénèmes par imperméabilité membranaire. Dans tout autre cas, un autre mécanisme de résistance est impliqué.

A des fins de vérification de la justesse des pronostics posés et la pertinence du postulat général précédemment énoncé :
- chaque souche a été testée en Western-blot en vue d'une détection de porines (en l'occurrence, des isoformes OmpF et OmpC), et
- pour certaines des souches testées, en particulier celles pour lesquelles il subsiste un doute quant au mécanisme de résistance développé (pronostic posé en contradiction avec les résultats de la détection des porines en Western-blot), un test de détection de carbapénémases au moyen du kit RAPIDEC® CARBA NP (bioMérieux, France) a également été réalisé.

S'agissant des résultats ainsi obtenus, la caractérisation des souches dites résistantes aux carbapénèmes par imperméabilité membranaire est établie, soit par l'absence de porines détectée en Western-blot, soit par une résistance aux carbapénèmes non liée à la présence de carbapénèmase vérifiée par une méthode moléculaire (PCR) et/ou par une méthode phénotypique (RAPIDEC® CARBA NP).

Les mesures de CMI_{IM/ETP} et les résultats de Western-blot ainsi obtenus, sont compilés dans le Tableau 3 ci-après.

**Tableau 3 (partie 1/2)**

| | ***E. coli 0506040*** (productrice de VIM-1) CMI_{PMBN} = 128 µg/mL | | | | | ***K. pneumoniae 1002015*** CMI_{PMBN} = 1024 µg/mL | | | | |
|---|---|---|---|---|---|---|---|---|---|---|
| [*PMBN*] | *0* | *CMI*/*10* | *CMI*/*8* | *CMI*/*5* | Vérifications | *0* | *CMI*/*10* | *CMI*/*8* | *CMI*/*5* | Vérifications |
| CMI_{IM} | 4 | 4 | 4 | 4 | Porine + | 2 | 0,5 | 0,25 | 0,25 | Porine-; Carbapénémases non détectées |
| CMI_{ETP} | 0,25 | 0,25 | 0,25 | 0,25 | | 32 | 4 | 2 | 1 | |
| | | | | | | | | | | |

| | ***S. marcescens 1008175*** (productrice d'IMP-1) CMI_{PMBN} = 1024 µg/mL | | | | | ***K. pneumoniae 1012335*** (productrice d'OXA-48) CMI_{PMBN} = 1024 µg/mL | | | | |
|---|---|---|---|---|---|---|---|---|---|---|
| [*PMBN*] | *0* | *CMIl10* | *CMI*/*8* | *CMI*/*5* | Vérifications | *0* | *CMI*/*10* | *CMI*/*8* | *CMI*/*5* | Vérifications |
| CMI_{IM} | 128 | 128 | 256 | 256 | Porine + | 2 | 2 | 2 | 2 | Porine + |
| CMI_{ETP} | 256 | 256 | 256 | 256 | | 2 | 4 | 4 | 4 | |
| | | | | | | | | | | |

| | ***K. pneumoniae 1104053*** (productrice d'OXA-48) CMI_{PMBN} = 1024 µg/mL | | | | | ***K. pneumoniae 1108001*** (productrice de KPC-2) CMI_{PMBN} = 1024 µg/mL | | | | |
|---|---|---|---|---|---|---|---|---|---|---|
| [*PMBN*] | *0* | *CMI*/*10* | *CMI*/*8* | *CMI*/*5* | Vérifications | *0* | *CMI*/*10* | *CMI*/*8* | *CMI*/*5* | Vérifications |
| CMI_{IM} | 2 | 2 | 2 | 2 | Porine + | 8 | 8 | 8 | 4 | Porine + |
| CMI_{ETP} | 4 | 4 | 4 | 4 | | 16 | 16 | 8 | 8 | |

| | ***E. aerogenes 1204048*** CMI_{PMBN} > 1024 µg/mL | | | | | ***K. pneumoniae 1108016*** (productrice de VIM-1) CMI_{PMBN} = 512 µg/mL | | | | |
|---|---|---|---|---|---|---|---|---|---|---|
| [*PMBN*] | *0* | *CMI*/*10* | *CMI*/*8* | *CMI*/*5* | Vérifications | *0* | *CMI*/*10* | *CMI*/*8* | *CMI*/*5* | Vérifications |
| CMI_{IM} | 16 | 2 | 1 | 0,5 | Porine - | 4 | 8 | 8 | 4 | Porine + |
| CMI_{ETP} | 32 | 4 | 2 | 2 | | 1 | 4 | 4 | 2 | |
| | | | | | | | | | | |

| | ***E. coli 1204043*** CMI_{PMBN} = 512 µg/mL | | | | | ***K. pneumoniae 1204034*** CMI_{PMBN} = 256 µg/mL | | | | |
|---|---|---|---|---|---|---|---|---|---|---|
| *West.blot* | *0* | *CMI*/*10* | *CMI*/*8* | *CMI*/*5* | Vérifications | *0* | *CMI*/*10* | *CMI*/*8* | *CMI*/*5* | Vérifications |
| CMI_{IM} | 8 | 2 | 1 | 1 | Porine - | 2 | 0,5 | 0,5 | 0,5 | Porine-; Carbapénémases non détectées |
| CMI_{ETP} | 32 | 1 | 2 | 1 | | 64 | 8 | 4 | 4 | |
| | | | | | | | | | | |

| | ***K. pneumoniae 1108007*** (productrice VIM-1) CMI_{PMBN} = 1024 µg/mL | | | | | ***C. freundii 1204047*** CMI_{PMBN} = 1024 µg/mL | | | | |
|---|---|---|---|---|---|---|---|---|---|---|
| [*PMBN*] | *0* | *CMI*/*10* | *CMI*/*8* | *CMI*/*5* | Vérifications | *0* | *CMI*/*10* | *CMI*/*8* | *CMI*/*5* | Vérifications |
| CMI_{IM} | 32 | 16 | 16 | 16 | Porine + | 1 | 2 | 1 | 1 | Porine +; Carbapénémases non détectées |
| CMI_{ETP} | 8 | 16 | 16 | 8 | | 32 | 8 | 8 | 4 | |
| | | | | | | | | | | |

| | ***E. aerogenes 9203076*** CMI_{PMBN} = 1024 µg/mL | | | | | ***E. cloacae 9304026*** CMI_{PMBN} > 1024 µg/mL | | | | |
|---|---|---|---|---|---|---|---|---|---|---|
| [*PMBN*] | *0* | *CMI*/*10* | *CMI*/*8* | *CMI*/*5* | Vérifications | *0* | *CMI*/*10* | *CMI*/*8* | *CMI*/*5* | Vérifications |
| CMI_{IM} | 8 | 2 | 2 | 2 | Porine - | 8 | 4 | 4 | 4 | Porine +; Carbapénémases non détectées |
| CMI_{ETP} | 32 | 4 | 4 | 2 | | 16 | 4 | 8 | 8 | |

**Tableau 3 (partie 2/2)**

| | ***E. aerogenes 9306071*** CMI_{PMBN} = 1024 µg/mL | | | | | ***E. cloacae 9402034*** CMI_{PMBN} > 1024 µg/mL | | | | |
|---|---|---|---|---|---|---|---|---|---|---|
| [*PMBN*] | *0* | *CMI*/*10* | *CMI*/*8* | *CMI*/*5* | Vérifications | *0* | *CMI*/*10* | *CMI*/*8* | *CMI*/*5* | Vérifications |
| CMI_{IM} | 8 | 4 | 4 | 2 | Porine-; Carbapénémases non détectées | 16 | 8 | 8 | 8 | Porine-; Carbapénémases non détectées |
| CMI_{ETP} | 32 | 4 | 4 | 4 | | 64 | 16 | 16 | 16 | |
| | | | | | | | | | | |

| | ***K. pneumoniae 1108002*** (productrice de KPC-3) CMI_{PMBN} > 1024 µg/mL | | | | | ***C. freundii 0403012*** CMI_{PMBN} = 256 µg/mL | | | | |
|---|---|---|---|---|---|---|---|---|---|---|
| [*PMBN*] | *0* | *CMI*/*10* | *CMI*/*8* | *CMI*/*5* | Vérifications | *0* | *CMI*/*10* | *CMI*/*8* | *CMI*/*5* | Vérifications |
| CMI_{IM} | 16 | 16 | 16 | 16 | Porine - | 4 | 2 | 1 | 1 | porine +; Carbapénémases non détectées |
| CMI_{ETP} | 16 | 64 | 64 | 64 | | 8 | 8 | 4 | 4 | |
| | | | | | | | | | | |

| | ***K. pneumoniae 0404024*** (productrice VIM-1) CMI_{PMBN} > 1024 µg/mL | | | | | ***E. cloacae 0901026*** (productrice KPC-2) CMI_{PMBN} = 1024 µg/mL | | | | |
|---|---|---|---|---|---|---|---|---|---|---|
| [*PMBN*] | *0* | *CMI*/*10* | *CMI*/*8* | *CMI*/*5* | Vérifications | *0* | *CMI*/*10* | *CMI*/*8* | *CMI*/*5* | Vérifications |
| CMI_{IM} | 64 | 64 | 64 | 64 | Porine + | 16 | 16 | 8 | 8 | Porine + |
| CMI_{ETP} | 128 | 32 | 32 | 16 | | 16 | 32 | 32 | 8 | |
| | | | | | | | | | | |

| | ***K. pneumoniae 0901027*** (productrice KPC-3) CMI_{PMBN} > 1024 µg/mL | | | | | ***E. cloacae 1008073*** (productrice VIM-1) CMI_{PMBN} = 1024 µg/mL | | | | |
|---|---|---|---|---|---|---|---|---|---|---|
| [*PMBN*] | *0* | *CMI*/*10* | *CMI*/*8* | *CMI*/*5* | Vérifications | *0* | *CMI*/*10* | *CMI*/*8* | *CMI*/*5* | Vérifications |
| CMI_{IM} | 8 | 16 | 8 | 16 | Porine + | 0,25 | 0,25 | 0,25 | 0,25 | Porine + |
| CMI_{ETP} | 16 | 64 | 64 | 64 | | 2 | 1 | 1 | 1 | |
| | | | | | | | | | | |

| | ***E. coli 1008077*** (productrice VIM-1) CMI_{PMBN} = 1024 µg/ mL | | | | | ***E. cloacae 1008079*** (productrice IMP-1) CMI_{PMBN} > 1024 µg/mL | | | | |
|---|---|---|---|---|---|---|---|---|---|---|
| [*PMBN*] | *0* | *CMI*/*10* | *CMI*/*8* | *CMI*/*5* | Vérifications | *0* | *CMI*/*10* | *CMI*/*8* | *CMI*/*5* | Vérifications |
| CMI_{IM} | 16 | 8 | 16 | 16 | Porine + | 2 | 4 | 2 | 2 | Porine + |
| CMI_{ETP} | 8 | 4 | 4 | 4 | | 4 | 8 | 8 | 8 | |
| | | | | | | | | | | |

| | ***E. cloacae 1008096*** (productrice VIM-1) CMI_{PMBN} = 1024 µg/mL | | | | | ***K. pneumoniae 1108006*** (productrice KPC-2) CMI_{PMBN} > 1024 µg/mL | | | | |
|---|---|---|---|---|---|---|---|---|---|---|
| [*PMBN*] | *0* | *CMI*/*10* | *CMI*/*8* | *CMI*/*5* | Vérifications | *0* | *CMI*/*10* | *CMI*/*8* | *CMI*/*5* | Vérifications |
| CMI_{IM} | 4 | 8 | 8 | 4 | Porine + | 32 | 32 | 64 | 32 | Porine + |
| CMI_{ETP} | 4 | 4 | 4 | 4 | | 64 | 256 | 256 | 256 | |
| | | | | | | | | | | |

| | ***E. cloacae 1101172*** (productrice NDM) CMI_{PMBN} = 1024 µg/mL | | | | | ***E. coli 1012292*** (productrice KPC) CMI_{PMBN} = 1024 µg/mL | | | | |
|---|---|---|---|---|---|---|---|---|---|---|
| [*PMBN*] | *0* | *CMI*/*10* | *CMI*/*8* | *CMI*/*5* | Vérifications | *0* | *CMI*/*10* | *CMI*/*8* | *CMI*/*5* | Vérifications |
| CMI_{IM} | 4 | 8 | 16 | 4 | Porine + | 8 | 4 | 4 | 1 | Porine + |
| CMI_{ETP} | 4 | 8 | 8 | 4 | | 8 | 4 | 4 | 2 | |

### a) Interprétation des résultats obtenus, évaluation des pronostics posés en application du postulat général

La notion de « fiabilité » utilisée ci-après, pour qualifier la pertinence de la méthode de discrimination selon l'invention et la justesse de l'interprétation des résultats, fait référence à une corrélation entre l'interprétation des mesures de CMI données par ladite méthode et les résultats d'une détection physique de porines et de carbapénémases. Il ne s'agit donc pas d'une fiabilité « absolue » mais d'une fiabilité relative, une « pseudo-fiabilité ».

Il faut aussi garder à l'esprit que la présence de porines dans la membrane des souches criblées apporte seulement une présomption quant à l'absence d'une résistance par imperméabilité membranaire, mais n'en procure pas une preuve incontestable (sauf à obtenir également la confirmation d'une présence suffisante de ces porines, aussi bien en nombre qu'en activité). Par contre, une absence de détection de carbapénémase permet une forte présomption de résistance par imperméabilité membranaire.

Malgré tout, la vérification par une détection physique de porines et de carbapénémases, ici mise en oeuvre, fournit un bon standard d'évaluation des performances et de la pertinence de la présente méthode de discrimination des mécanismes de résistance aux carbapénèmes développés par les bactéries. On estime ainsi que la caractérisation des souches dites résistantes aux carbapénèmes par imperméabilité membranaire est établie, soit par l'absence de porines détectée en Western-blot, soit par une résistance aux carbapénèmes non liée à la présence de carbapénèmase vérifiée par une méthode moléculaire (PCR) et/ou par une méthode phénotypique (RAPIDEC® CARBA NP).

Au vu des résultats présentés dans le Tableau 3, il est constaté ce qui suit :
- Sur les 168 valeurs de CMI_{IM} et CMI_{ERT}, mesurées en présence de PMBN à des concentrations non-inhibitrices, 133 sont conformes au postulat général précédemment énoncé. Une interprétation des résultats obtenus par la méthode de discrimination selon l'invention, basée sur ce postulat général, présente une corrélation de l'ordre de 79 % avec les vérifications effectuées en Western-blot, et éventuellement avec le test RAPIDEC ® CARBA NP. La corrélation est d'environ 81 % avec l'imipénème et d'environ 77 % avec l'ertapénème.
   A cet égard, il convient de noter que pour les souches *C. freundii* 1204047, *E. cloacae* 9304026 et *C. freundii* 0403012, compte tenu de l'absence de carbapénémase détectée avec le test RAPIDEC® CARBA NP, il existe une forte présomption quant à une résistance par imperméabilité membranaire. Les porines détectées en Western-blot semblent en effet être qualitativement et/ou quantitativement non-fonctionnelles.
- Sur ces 168 valeurs, 87 ne correspondent pas à une diminution de CMI_{IM/ETP} et laissent supposer un mécanisme de résistance autre qu'une imperméabilité membranaire, pour les souches concernées. Les vérifications effectuées ont permis de confirmer l'exactitude de 77 de ces pronostics. Ainsi avec cette méthode, un pronostic négatif quant à une résistance par imperméabilité membranaire offre une fiabilité de l'ordre de 89 % (87 % avec l'imipénème et 90 % avec l'ertapénème).
- Sur ces 168 valeurs, 81 valeurs correspondent à une diminution CMI_{IM/ETP} et laissent supposer une résistance par imperméabilité membranaire pour les souches concernées. Les vérifications effectuées ont permis de confirmer l'exactitude de 56 de ces pronostics. Avec la méthode décrite, un pronostic positif quant à une résistance par imperméabilité membranaire présente donc une fiabilité de l'ordre 69 % (73 % avec l'imipénème et 66 % avec l'ertapénème).

Ainsi, une méthode de discrimination selon l'invention, à laquelle est appliquée une règle d'interprétation des valeurs de CMI de carbapénème indicateur relevées, conforme au postulat général précédemment énoncé, devrait pouvoir offrir une fiabilité générale de l'ordre 79 %. Cette fiabilité devrait être de l'ordre de 89 % lorsqu'une résistance autre qu'une imperméabilité membranaire est pronostiquée. Lorsqu'une imperméabilité membranaire est pronostiquée, il est alors recommandé d'entreprendre des tests de confirmation complémentaires.

### b) Autre mode d'interprétation des résultats obtenus, évaluation des pronostics posés en application d'un postulat affiné

Un deuxième mode d'interprétation des résultats compilés dans le Tableau 3 est ici proposé. Le système de pronostic est basé sur le postulat « affiné » suivant : Une diminution de CMI_{IM/ETP}, en présence de PMBN, d'au moins un facteur 4 (c'est-à-dire avec un écart de CMI de 2 dilutions de raison 2), est annonciateur d'une résistance aux carbapénèmes par imperméabilité membranaire. Sinon, un autre mécanisme de résistance est impliqué.

En considération de ce postulat affiné et au vu des résultats présentés dans le Tableau 3, il est constaté ce qui suit :
- Sur les 168 valeurs de CMI_{IM} et CMI_{ERT}, mesurées en présence de PMBN à des concentrations non-inhibitrices, 140 sont conformes au postulat affiné précédemment énoncé. Une interprétation des résultats obtenus par la méthode de discrimination selon l'invention, basée sur ce postulat général, présente une corrélation de l'ordre de 83 % avec les vérifications effectuées en Western-blot, et éventuellement avec le test RAPIDEC® CARBA NP. La corrélation est d'environ 81 % avec l'imipénème et d'environ 86 % avec l'ertapénème.
- Sur ces 168 valeurs, 120 ne correspondent pas à une diminution de CMI_{IM/ETP} d'au moins un facteur 4 et laissent supposer un mécanisme de résistance autre qu'une imperméabilité membranaire, pour les souches concernées. Les vérifications effectuées ont permis de confirmer l'exactitude de 92 de ces pronostics. Avec cette méthode, un pronostic négatif quant à une résistance par imperméabilité membranaire présente donc une fiabilité de l'ordre de 81 % (77 % avec l'imipénème et 85 % avec l'ertapénème).
- Sur ces 168 valeurs, 48 correspondent à une diminution de CMI_{IM/ETP} d'au moins un facteur 4 et laissent supposer une résistance par imperméabilité membranaire, pour les souches concernées. Les vérifications effectuées ont permis de confirmer l'exactitude de 43 de ces pronostics. Ainsi avec cette méthode, un pronostic positif quant à une résistance par imperméabilité membranaire présente une fiabilité de l'ordre de 90 % (95 % avec l'imipénème et 86 % avec l'ertapénème).

Ainsi, une méthode de discrimination selon l'invention, à laquelle est appliquée une règle d'interprétation des valeurs de CMI de carbapénème indicateur relevées, conforme au postulat affiné précédemment énoncé, devrait présenter une fiabilité globale améliorée, qui serait maintenant de l'ordre 83 %.

Cette amélioration est profitable aux pronostics positifs pour une imperméabilité membranaire, dont la fiabilité passe de 69 % à 90 %. Elle est apparemment obtenue au détriment des pronostics négatifs pour une imperméabilité membranaire, dont la fiabilité précédemment annoncée à 89 % recule légèrement et est maintenant de 81 %.

### c) Remarques complémentaires

Au vu des deux postulats précédents, une règle d'interprétation intermédiaire serait susceptible de permettre d'améliorer davantage la fiabilité de la présente méthode, à savoir :
- une diminution de CMI_{IM/ETP} en présence de PMBN, d'au moins un facteur 4 (c'est-à-dire avec un écart de CMI de 2 dilutions de raison 2), est annonciateur d'une résistance aux carbapénèmes par imperméabilité membranaire,
- une absence de diminution de CMI_{IM/ETP} en présence de PMBN est annonciateur d'un mécanisme de résistance autre qu'une imperméabilité membranaire,
- une diminution de CMI_{IM/ETP} en présence de PMBN, d'un facteur inférieur à 4, est annonciateur d'un mécanisme de résistance par imperméabilité membranaire, il est néanmoins recommandé de procéder à des tests complémentaires.

Par ailleurs, quoique moindres avec l'imipénème, les performances constatées avec l'imipénème et l'ertapénème, dans le cadre de la mise en oeuvre de la méthode de discrimination selon l'invention, sont très similaires et comparables. Ceci laisse présager la possibilité d'utiliser d'autres carbapénèmes à titre de carbapénèmes indicateurs.

Les données précédentes démontrent la pertinence de la présente méthode de discrimination du mécanisme de résistance aux carbapénèmes développé par une bactérie. Une certaine modularité est autorisée quant au choix du carbapénème indicateur à utiliser, quant à la concentration non-inhibitrice de PMBN à appliquer.

Une certaine modularité est également autorisée quant à l'interprétation des variations de CMI constatées, en fonction du carbapénème indicateur et de la concentration non-inhibitrice de PMBN appliqués, et éventuellement selon le caractère à mettre en évidence en priorité (résistance par imperméabilité membranaire ou autre mécanisme) et le niveau de fiabilité recherchée pour cette mise en évidence.

### 3. Simplification et optimisation de la méthode selon l'invention

En vue de simplifier la mise en oeuvre de cette méthode, une recherche de généralisation a été entreprise pour identifier une(des) concentration(s) non-inhibitrice(s) de PMBN « standard(s) », pouvant être avantageusement appliquée(s) à un grand nombre de bactéries. La détermination de telles concentrations standards permettraient d'éviter d'avoir à évaluer, pour chaque souche bactérienne à tester, son niveau de sensibilité à la PMBN.

Au vu des CMI_{PMBN} précédemment identifiées, très majoritairement bien supérieures à 300 µg/mL, les concentrations standards suivantes ont été retenues et appliquées aux souches bactériennes à tester : 50, 100, 150, 200 et 300 µg/mL.

La validation de ces concentrations standards de PMBN a été réalisée avec des souches bactériennes, issues de collections appartenant aux titulaires du présent Titre de propriété industrielle, et exprimant un phénotype de perméabilité membranaire, « porine + » ou « porine - ».

A titre de carbapénèmes indicateurs, en plus de l'imipénème (IM) et l'ertapénème (ETP), le méropénème (MP) a également été testé. A l'instar des deux autres antibiotiques, le méropénème a été utilisé sous la forme de bandelettes Etest® (Etest® Meropenem MP 32 de la société bioMérieux, France).

Les méthodes utilisées pour réaliser les nouveaux milieux de culture et les tests de sensibilité aux antibiotiques sont identiques à celles précédemment décrites.

Le Tableau 4 ci-après compile les résultats obtenus dans une première série de mesures où seules des souches « porines - » sont testées (les CMI et les concentrations en PMBN y sont exprimées en µg/mL).

**Tableau 4**

| | ***E. aerogenes* EA 5** (porine -) | | | | | ***E. aerogenes* EA 27** (porine -) | | | | |
|---|---|---|---|---|---|---|---|---|---|---|
| [*PMBN*] | *0* | *50* | *100* | *150* | *200* | *0* | *50* | *100* | *150* | *200* |
| CMI_{IM} | 6 | 4 | 4 | 4 | 3 | 12 | 6 | 3 | 3 | 3 |
| CMI_{ETP} | 6 | 2 | 2 | 1,5 | 1 | > 32 | 4 | 1,5 | 0,75 | 0,19 |
| CMI_{MP} | 3 | 2 | 1,5 | 0,75 | 0,75 | 4 | 0,75 | 0,5 | 0,19 | 0,094 |
| | | | | | | | | | | |

| | ***K. pneumoniae* KP 55** (porine -) | | | | | ***K. pneumoniae* KP 63** (porine -) | | | | |
|---|---|---|---|---|---|---|---|---|---|---|
| *[PMBN]* | *0* | *50* | *100* | *150* | *200* | *0* | *50* | *100* | *150* | *200* |
| CMI_{IM} | 0,38 | 0,25 | 0,25 | 0,25 | 0,19 | 0,25 | 0,25 | 0,125 | 0,125 | 0,125 |
| CMI_{ETP} | 0,38 | 0,25 | 0,19 | 0,125 | 0,064 | 1 | 0,50 | 0,38 | 0,19 | 0,125 |
| CMI_{MP} | 0,19 | 0,125 | 0,064 | 0,064 | 0,047 | 0,25 | 0,25 | 0,094 | 0,064 | 0,023 |
| | | | | | | | | | | |

| | ***E. coli* SAB 100** (porine -) | | | | | ***E. coli* SAB 108** (porine -) | | | | |
|---|---|---|---|---|---|---|---|---|---|---|
| [*PMBN*] | *0* | *50* | *100* | *150* | *200* | *0* | *50* | *100* | *150* | *200* |
| CMI_{IM} | 0,5 | 0,38 | 0,25 | 0,19 | 0,19 | 0,75 | 0,38 | 0,25 | 0,25 | 0,19 |
| CMI_{ETP} | 1,5 | 0,19 | 0,19 | 0,125 | 0,125 | 4 | 0,25 | 0,19 | 0,19 | 0,19 |
| CMI_{MP} | 0,125 | 0,094 | 0,047 | 0,032 | 0,032 | 2 | 0,125 | 0,125 | 0,094 | 0,094 |
| | | | | | | | | | | |

| | ***E. coli* 1204043** (porine -) | | | | | **E. aerogenes 9203076** (porine -) | | | | |
|---|---|---|---|---|---|---|---|---|---|---|
| [*PMBN*] | *0* | *50* | *100* | *150* | *200* | *0* | *50* | *100* | *150* | *200* |
| CMI_{IM} | 24 | - | - | 1 | 0,25 | 6 | - | - | 3 | 3 |
| CMI_{ETP} | > 32 | - | - | 0,38 | 0,19 | 24 | - | - | 3 | 1,5 |
| CMI_{MP} | 3 | - | - | 0,064 | 0,008 | 4 | - | - | 0,5 | 0,5 |
| | | | | | | | | | | |

| | ***K. pneumoniae* 1002015** (porine -) | | | | | ***K. pneumoniae* 1204034** (porine -) | | | | |
|---|---|---|---|---|---|---|---|---|---|---|
| [*PMBN*] | *0* | *50* | *100* | *150* | *200* | *0* | *50* | *100* | *150* | *200* |
| CMI_{IM} | 0,5 | - | - | 0,38 | 0,25 | 2 | - | - | 0,38 | 0,38 |
| CMI_{ETP} | 4 | - | - | 0,5 | 0,38 | > 32 | - | - | 1,5 | 1 |
| CMI_{MP} | 2 | - | - | 0,19 | 0,19 | 12 | - | - | 0,5 | 0,25 |

Le Tableau 4bis ci-après compile les résultats obtenus dans une deuxième série de tests, dans laquelle ont été testées aussi bien des souches bactériennes ayant une imperméabilité membranaire avérée que des souches productrices de carbapénémases.

**Tableau 4bis (1/2)**

| | ***K. pneumoniae 1204035*** (imperméabilité membranaire) | | | | ***K. pneumoniae 1204036*** (imperméabilité membranaire) | | | |
|---|---|---|---|---|---|---|---|---|
| [*PMBN*] | *0* | *100* | *200* | *300* | *0* | *100* | *200* | *300* |
| CMI_{IM} | 1,5 | 0,75 | 0,5-0,75 | 0,5-1 | 2 | 2 | 2 | 1,5 |
| CMI_{ETP} | >32 | 8 | 8-12 | 6 | >32 | >32 | >32 | >32 |
| CMI_{MP} | 8-12 | 2 | 1,5 | 1,5 | 32 | 16 | 6 | 4 |
| | | | | | | | | |

| | ***E. cloacae 1204051*** (imperméabilité membranaire) | | | | ***E. cloacae 1204052*** (imperméabilité membranaire) | | | |
|---|---|---|---|---|---|---|---|---|
| [*PMBN*] | *0* | *100* | *200* | *300* | *0* | *100* | *200* | *300* |
| CMI_{IM} | 1,5 | 1,5 | 0,75 | 0,75 | 3 | 3 | 2 | 1,5 |
| CMI_{ETP} | >32 | 12 | 4 | 3 | >32 | 12 | 8 | 3 |
| CMI_{MP} | 4 | 2 | 0,25 | 0,25 | 6 | 0,75 | 1,5 | 0,5 |
| | | | | | | | | |

| | ***E. cloacae 1204053*** (imperméabilité membranaire) | | | | ***E. cloacae 1204056*** (imperméabilité membranaire) | | | |
|---|---|---|---|---|---|---|---|---|
| [*PMBN*] | *0* | *100* | *200* | *300* | *0* | *100* | *200* | *300* |
| CMI_{IM} | 3 | 3 | 3 | 3 | 6 | 2 | 1,5 | 1 |
| CMI_{ETP} | >32 | >32 | >32 | 4 | >32 | 12 | 3 | 1 |
| CMI_{MP} | 4 | 2 | 2 | 1,5 | 12 | 2 | 1 | 0,75 |
| | | | | | | | | |

| | ***E. cloacae 1204058*** (imperméabilité membranaire) | | | | ***K. pneumoniae 1204039*** (imperméabilité membranaire) | | | |
|---|---|---|---|---|---|---|---|---|
| [*PMBN*] | *0* | *100* | *200* | *300* | *0* | *100* | *200* | *300* |
| CMI_{IM} | >32 | 12 | 4 | 3 | 0,38 | 0,38 | 0,38 | 0,38 |
| CMI_{ETP} | >32 | 8-12 | - | 0,75 | 2 | 1,5 | 1,5 | 1 |
| CMI_{MP} | >32 | 2 | 0,75 | 0,25 | 0,75 | 0,5 | 0.38 | 0,38 |
| | | | | | | | | |

| | ***E. aerogenes 1204050*** (imperméabilité membranaire) | | | | ***E. cloacae 1204153*** (imperméabilité membranaire) | | | |
|---|---|---|---|---|---|---|---|---|
| [*PMBN*] | *0* | *100* | *200* | *300* | *0* | *100* | *200* | *300* |
| CMI_{IM} | >32 | >32 | >32 | >32 | 4 | 4 | 2 | 4 |
| CMI_{ETP} | >32 | >32 | >32 | >32 | >32 | 8 | 4 | 3 |
| CMI_{MP} | >32 | 2-4 | 1,5-2 | 1 | 4 | 0,75-1 | 1 | 0,75 |
| | | | | | | | | |

| | ***E. cloacae 9402034*** (imperméabilité membranaire) | | | | ***E. aerogenes 9306071*** (imperméabilité membranaire) | | | |
|---|---|---|---|---|---|---|---|---|
| [*PMBN*] | *0* | *100* | *200* | *300* | *0* | *100* | *200* | *300* |
| CMI_{IM} | >32 | 8 | 12 | 12 | 12 | 6 | 3 | 2 |
| CMI_{ETP} | >32 | >32 | 12 | 12 | >32 | 12 | 8 | 4 |
| CMI_{MP} | 16 | 2 | 2 | 3 | 4 | 1,5 | 1,5 | 0,5 |

**Tableau 4bis (2/2)**

| | ***E. aerogenes 9203076*** (imperméabilité membranaire) | | | | ***K. pneumoniae 1204034*** (imperméabilité membranaire) | | | |
|---|---|---|---|---|---|---|---|---|
| [*PMBN*] | *0* | *100* | *200* | *300* | *0* | *100* | *200* | *300* |
| CMI_{IM} | 8 | 16 | 4 | 4 | 3 | 0,5 | 0,38 | 0,19 |
| CMI_{ETP} | >32 | 16 | 12 | 4 | >32 | 12 | 3 | 2 |
| CMI_{MP} | 4 | 2 | 1,5 | 0,5-0,75 | 16 | 1,5 | 0,75 | 0,38 |
| | | | | | | | | |

| | ***E. coli 1204043*** (imperméabilité membranaire) | | | | ***E. aerogenes 1204048*** (imperméabilité membranaire) | | | |
|---|---|---|---|---|---|---|---|---|
| [*PMBN*] | *0* | *100* | *200* | *300* | *0* | *100* | *200* | *300* |
| CMI_{IM} | >32 | 6-8 | 3-6 | 3 | >32 | 12 | 6 | 3 |
| CMI_{ETP} | >32 | 6 | 2-4 | 1,5 | >32 | 16 | 16 | 8 |
| CMI_{MP} | 4 | 0,75 | 0,38 | 0,19 | >32 | 1,5 | 1 | 0,5-1 |
| | | | | | | | | |

| | ***K. pneumoniae 1012142*** (productrice de KPC-2) | | | | ***E. cloacae 1008080*** (productrice de IMP) | | | |
|---|---|---|---|---|---|---|---|---|
| [*PMBN*] | *0* | *100* | *200* | *300* | *0* | *100* | *200* | *300* |
| CMI_{IM} | >32 | >32 | >32 | >32 | 1 | 12 | 3 | 6 |
| CMI_{ETP} | 32 | >32 | >32 | >32 | 16 | 24 | >32 | 12-24 |
| CMI_{MP} | >32 | >32 | >32 | >32 | 2 | 8 | 6 | 4-6 |
| | | | | | | | | |

| | ***E. coli 1008078*** (productrice de VIM) | | | | ***K. pneumoniae 1108003*** (productrice de KPC-3) | | | |
|---|---|---|---|---|---|---|---|---|
| [*PMBN*] | *0* | *100* | *200* | *300* | *0* | *100* | *200* | *300* |
| CMI_{IM} | 0,38 | 1,5 | 0,75 | 0,38 | 6 | 8 | 12 | 12 |
| CMI_{ETP} | 2 | 0,75 | 0,5 | 0,38 | 8 | 24 | >32 | >32 |
| CMI_{MP} | 0,5 | 0,5 | 0,25 | 0,25 | 6 | >32 | >32 | 4 |
| | | | | | | | | |

| | ***K. pneumoniae 1103183*** (productrice de NDM) | | | | ***E. cloacae 1109137*** (productrice d'OXA-48) | | | |
|---|---|---|---|---|---|---|---|---|
| [*PMBN*] | *0* | *100* | *200* | *300* | *0* | *100* | *200* | *300* |
| CMI_{IM} | 24 | >32 | >32 | 6 | 1,5 | 4 | 2 | 1,5 |
| CMI_{ETP} | >32 | >32 | 32 | 32 | 24 | - | 12 | >32 |
| CMI_{MP} | >32 | >32 | 24 | >32 | 3 | 3 | 1,5 | 2 |

| | ***E. cloacae 1008074*** (productrice de VIM) | | | | | | | |
|---|---|---|---|---|---|---|---|---|
| [*PMBN*] | *0* | *100* | *200* | *300* | | | | |
| CMI_{IM} | 3 | 3 | 8 | 6 | | | | |
| CMI_{ETP} | 0,38 | 0,5 | 0,5 | 0,5 | | | | |
| CMI_{MP} | 0,38 | 1,5 | 1 | 1,5 | | | | |

Il ressort des Tableaux 4 et 4bis, que les résultats obtenus montrent une bonne homogénéité entre les variations de CMI obtenues avec les trois carbapénèmes testés (près de 90 % de concordance entre les variations). Cette bonne homogénéité confirme que la mise en oeuvre de la méthode de discrimination selon l'invention peut être généralisée à d'autres carbapénèmes. L'imipénème (IM) présente toutefois des performances quelque peu moindres.

Les résultats les plus fiables sont obtenus aux concentrations non-inhibitrices de PMBN les plus élevées, et plus encore avec le méropénème (MP) et l'ertapénème (ETP).

Notamment, en appliquant la règle d'interprétation « affinée » précédemment énoncée, pour des concentrations non-inhibitrices de PMBN, de 150 à 300 µg/mL, le méropénème a conduit à l'obtention de pronostics à 81,8 % attendus, et l'ertapénème 81,5 %.

### 4. Méthodes d'évaluation de la sensibilité aux antibiotiques autres que la méthode Etest® classique, et validation d'autres modes de réalisation

Dans les exemples précédents, la méthode de discrimination selon l'invention a été mise en oeuvre avec une étape d'évaluation de la sensibilité des bactéries au carbapénème indicateur réalisée au moyen d'une méthode de diffusion de l'antibiotique sur un milieu de culture (avec ou sans PMBN) solide, et utilisant des bandelettes imprégnées d'un gradient d'un carbapénème indicateur (méthode E-test®).

D'autres variantes de réalisation de cette étape d'évaluation de la sensibilité des bactéries au carbapénème indicateur ont également été testées, à savoir :
- une méthode de diffusion de l'antibiotique sur un milieu de culture solide sans PMBN, et utilisant des bandelettes imprégnées à la fois d'un gradient de carbapénème et d'une quantité standard de PMBN ;
- une méthode automatisée de micro-dilution en milieu liquide, effectuée en adaptant la technologie et l'instrumentation VITEK® 2 AST (bioMérieux, France).

### a) Diffusion du carbapénème indicateur et de PMBN à partir d'une même bandelette Etest®.

Dans ce mode de réalisation particulier de l'invention, la PMBN n'est pas initialement présente dans le milieu de culture mais, tout comme le carbapénème indicateur, elle est apportée de manière extemporanée, par diffusion progressive à partir d'une bandelette imprégnée.

Les bandelettes utilisées sont imprégnées à la fois d'un carbapénème indicateur et de PMBN. Ces bandelettes ont été préparées à partir de bandelette Etest® de la société bioMérieux (France) :
- Etest® Imipenem IM 32,
- Etest® Ertapenem ETP 32, et
- Etest® Meropenem MP 32,
que l'on a imprégnées d'une solution de PMBN.

Plusieurs solutions d'imprégnation, de concentrations différentes en PMBN (100, 200 et 300 µg/mL) ont été testées.

Ces bandelettes qui cumulent PMBN et carbapénème indicateur sont alors testées pour leur capacité à pouvoir mettre en oeuvre la méthode de discrimination selon l'invention. Pour ce faire, des souches bactériennes de phénotype de résistance connu :
- imperméabilité membranaire, ou
- production de carbapénémase ;
ont été utilisées comme modèles.

Le Tableau 5 ci-après résume les résultats obtenus. Ces résultats y sont exprimés en [nombre de souches testées positivement pour une résistance par imperméabilité membranaire, sur le nombre de souches testées] et ce, en fonction du carbapénème indicateur utilisé et de la concentration en PMBN de la solution d'imprégnation des bandelettes Etest®.

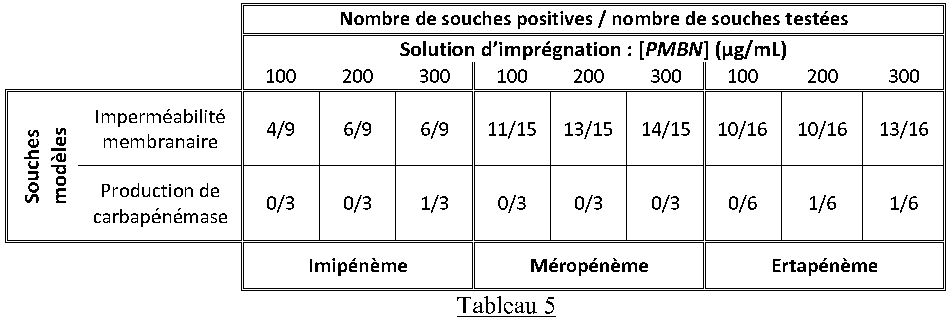

Les résultats ainsi obtenus permettent de valider ce mode particulier de mise en oeuvre de la méthode de discrimination selon l'invention, dans lequel PMBN et carbapénème indicateur sont apportés de manière extemporanée au milieu de culture solide, au moyen d'une bandelette Etest®.

### b) Evaluation de la susceptibilité au carbapénème indicateur par micro-dilution en milieu liquide.

Dans ce mode particulier de réalisation de l'invention, l'évaluation de la susceptibilité de la souche testée au carbapénème indicateur est effectuée au moyen d'une méthode de micro-dilution. Le milieu de culture n'est donc plus un milieu solide, mais un ensemble de milieux liquides, de différentes concentrations en carbapénème indicateur.

En l'occurrence, les inventeurs ont choisi d'adapter la technologie et l'instrumentation VITEK® 2 AST (bioMérieux, France), à la présente méthode de discrimination. Ce faisant, l'étape d'évaluation de la sensibilité des souches testées au carbapénème indicateur se trouve avantageusement automatisée.

Pour ce faire, les cartes VITEK® 2 AST comprennent un ensemble de cupules remplies d'un milieu de culture liquide comprenant une gamme de concentrations d'un carbapénème indicateur, et une concentration fixe non-inhibitrice de PMBN.

Le Tableau 6 ci-après résume les résultats obtenus. Ces résultats y sont exprimés en [nombre de souches testées positivement pour une résistance par imperméabilité membranaire, sur le nombre de souches testées] et ce, en fonction du carbapénème indicateur et de la concentration non-inhibitrice de PMBN utilisés.

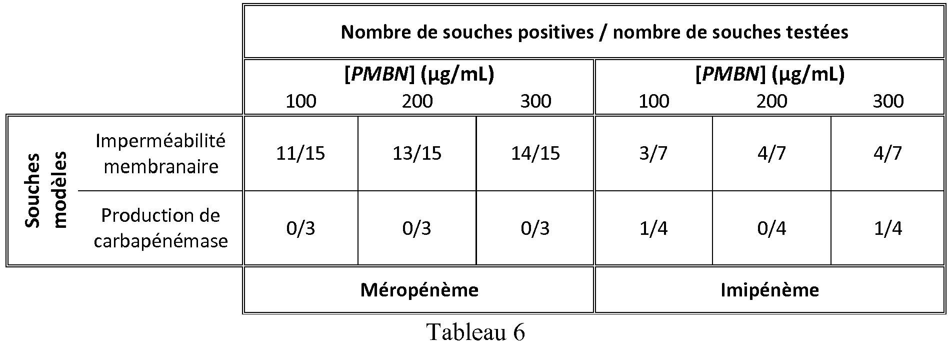

Ces premiers résultats confirment que l'étape d'évaluation de la sensibilité au carbapénème indicateur peut être effectuée par micro-dilution en milieu liquide, notamment en revisitant la technologie et l'instrumentation VITEK® 2 AST (bioMérieux, France).

## Revendications

1. Méthode de discrimination de bactéries résistantes aux carbapénèmes selon qu'elles développent ou non une résistance par imperméabilité membranaire, ladite méthode comprenant les étapes suivantes :
- disposer d'une souche bactérienne résistante à au moins un carbapénème,
- évaluer la sensibilité de ladite souche bactérienne à l'égard d'au moins un carbapénème indicateur ; d'une part, en absence de polymyxine B nonapeptide (PMBN) et, d'autre part, en présence de PMBN à une concentration inférieure à la concentration minimale inhibitrice (CMI) de la PMBN pour ladite souche bactérienne, dite concentration non-inhibitrice de PMBN ;
- poser un pronostic de résistance par imperméabilité membranaire dans le cas où, la présence de PMBN permet d'accroitre de manière significative la sensibilité de ladite souche bactérienne à l'égard d'au moins un carbapénème indicateur utilisé.

2. Méthode selon la revendication 1, **caractérisée en ce que** chaque carbapénème indicateur est choisi parmi : l'imipénème, l'ertapénème et le méropénème.

3. Méthode selon la revendication 1 ou 2, **caractérisée** en ce ladite concentration non-inhibitrice de PMBN est comprise entre de l'ordre de un dixième (1/10) et de l'ordre de un demi (1/2) de la CMI de la PMBN pour ladite souche bactérienne.

4. Méthode selon l'une quelconque des revendications précédentes, **caractérisée en ce que** ladite concentration non-inhibitrice de PMBN est comprise entre de l'ordre de 25 µg/mL et de l'ordre 500 µg/mL.

5. Méthode selon l'une quelconque des revendications précédentes, **caractérisée en ce que** l'accroissement de la sensibilité de ladite souche bactérienne à un carbapénème indicateur est significatif dès lors qu'une diminution de CMI d'au moins un facteur 2 est constatée.

6. Méthode selon l'une quelconque des revendications précédentes, **caractérisée en ce que** l'évaluation de la sensibilité de la souche bactérienne à chaque carbapénème indicateur est réalisée au moyen d'une méthode de diffusion sur milieu solide.

7. Méthode selon la revendication 6, **caractérisée en ce que** ladite méthode de diffusion sur milieu solide, dans laquelle chaque carbapénème indicateur est utilisé sous la forme d'une bandelette de gradient d'antibiotique.

8. Méthode selon la revendication 6 ou 7, **caractérisée en ce que** la PMBN est initialement présente dans ledit milieu solide à une concentration non-inhibitrice.

9. Méthode selon la revendication 6 ou 7, **caractérisée en ce que** la PMBN est apportée audit milieu solide de façon extemporanée et concomitante avec le(s) carbapénème(s) indicateur(s).

10. Méthode selon l'une quelconque des revendications 1 à 5, **caractérisée en ce que** l'évaluation de la sensibilité de la souche bactérienne à l'égard de chaque carbapénème indicateur est réalisée au moyen d'une méthode de micro-dilution en milieu liquide.

## Patentansprüche

1. Unterscheidungsverfahren für Carbapenem-resistente Bakterien je nachdem ob sie eine Resistenz durch Membranundurchlässigkeit entwickeln oder nicht, wobei das Verfahren die folgenden Schritte umfasst:
Bereitstellen eines Bakterienstamms, der gegenüber mindestens einem Carbapenem resistent ist,
Auswerten der Empfindlichkeit des Bakterienstamms in Bezug auf mindestens ein Indikator-Carbapenem; einerseits in Abwesenheit von Polymyxin-B-Nonapeptid (PMBN) und andererseits in Anwesenheit von PMBN bei einer Konzentration unterhalb der minimalen inhibitorischen Konzentration (MIC) des PMBN für den Bakterienstamm, welche als nicht-inhibitorische PMBN-Konzentration bezeichnet wird;
Erstellen einer Prognose der Resistenz durch Membranundurchlässigkeit in dem Fall, in dem es die Anwesenheit von PMBN ermöglicht, die Empfindlichkeit des Bakterienstamms in Bezug auf mindestens ein verwendetes Indikator-Carbapenem signifikant zu erhöhen.

2. Verfahren gemäß Anspruch 1, **dadurch gekennzeichnet, dass** jedes Indikator-Carbapenem ausgewählt ist aus: Imipenem, Ertapenem und Meropenem.

3. Verfahren gemäß Anspruch 1 oder 2, **dadurch gekennzeichnet, dass** die nicht-inhibitorische PMBN-Konzentration zwischen etwa einem Zehntel (1/10) und etwa der Hälfte (1/2) der MIC des PMBN für den Bakterienstamm liegt.

4. Verfahren gemäß einem der vorhergehenden Ansprüche, **dadurch gekennzeichnet, dass** die nicht-inhibitorische PMBN-Konzentration zwischen etwa 25 µg/ml und etwa 500 µg/ml liegt.

5. Verfahren gemäß einem der vorhergehenden Ansprüche, **dadurch gekennzeichnet, dass** die Erhöhung der Empfindlichkeit des Bakterienstamms gegenüber einem Indikator-Carbapenem signifikant ist, wenn eine Abnahme der MIC um mindestens einen Faktor 2 festgestellt wird.

6. Verfahren gemäß einem der vorhergehenden Ansprüche, **dadurch gekennzeichnet, dass** die Auswertung der Empfindlichkeit des Bakterienstamms gegenüber jedem Indikator-Carbapenem mittels eines Diffusionsverfahrens auf festem Medium durchgeführt wird.

7. Verfahren gemäß Anspruch 6, **dadurch gekennzeichnet, dass** bei dem Diffusionsverfahren auf festem Medium jedes Indikator-Carbapenem in Form eines Antibiotika-Gradientenstreifens verwendet wird.

8. Verfahren gemäß Anspruch 6 oder 7, **dadurch gekennzeichnet, dass** das PMBN anfangs bei einer nicht-inhibitorischen Konzentration in dem festen Medium vorliegt.

9. Verfahren gemäß Anspruch 6 oder 7, **dadurch gekennzeichnet, dass** das PMBN unmittelbar vor dem Gebrauch und gleichzeitig mit dem Indikator-Carbapenem (den Indikator-Carbapenemen) in das feste Medium eingebracht wird.

10. Verfahren gemäß einem der Ansprüche 1 bis 5, **dadurch gekennzeichnet, dass** die Auswertung der Empfindlichkeit des Bakterienstamms in Bezug auf jedes Indikator-Carbapenem mittels eines Mikroverdünnungsverfahrens in flüssigem Medium durchgeführt wird.

## Claims

1. A method for discriminating carbapenem-resistant bacteria according to whether or not they develop resistance by membrane impermeability, said method comprising the following steps:
- providing a bacterial strain that is resistant to at least one carbapenem,
- evaluating the sensitivity of said bacterial strain with regard to at least one indicator carbapenem, on the one hand in the absence of polymyxin B nonapeptide (PMBN) and, on the other hand, in the presence of PMBN at a non-inhibitory concentration which is lower than the minimum inhibitory concentration (MIC) of PMBN for said bacterial strain,
- establishing a prognosis of resistance by membrane impermeability in the case where the presence of PMBN makes it possible to significantly increase the sensitivity of said bacterial strain with regard to at least one indicator carbapenem used.

2. The method according to claim 1, **characterized in that** each indicator carbapenem is chosen from: imipenem, ertapenem and meropenem.

3. The method according to claim 1 or 2, **characterized in that** said non-inhibitory concentration of PMBN is between about one tenth (1/10) and about half (1/2) of the MIC of PMBN for said bacterial strain.

4. The method according to any one of the preceding claims, **characterized in that** said non-inhibitory concentration of PMBN is between about 25 µg/ml and about 500 µg/ml.

5. The method according to any one of the preceding claims, **characterized in that** the increase in the sensitivity of said bacterial strain to an indicator carbapenem is significant provided that a decrease in MIC of at least a factor of 2 is noted.

6. The method according to any one of the preceding claims, **characterized in that** the evaluation of the sensitivity of the bacterial strain to each indicator carbapenem is carried out by means of a method of diffusion on solid medium.

7. The method according to claim 6, wherein each indicator carbapenem is used in the form of a strip of antibiotic gradient.

8. The method according to claim 6 or 7, **characterized in that** the PMBN is initially present in said solid medium at a non-inhibitory concentration.

9. The method according to claim 6 or 7, **characterized in that** the PMBN is provided to said solid medium extemporaneously and concomitantly with the indicator carbapenem(s).

10. The method according to any one of the claims 1 to 5, **characterized in that** the evaluation of the sensitivity of the bacterial strain with regard to each indicator carbapenem is carried out by means of a method of microdilution in liquid medium.
